(19) **Europäisches Patentamt**
**European Patent Office**
**Office européen des brevets**

(11) **EP 2 309 257 A1**

(12) **EUROPEAN PATENT APPLICATION**

(43) Date of publication:
**13.04.2011 Bulletin 2011/15**

(51) Int Cl.:
**G01N 23/04** *(2006.01)* **G01V 5/00** *(2006.01)*
**A61B 6/03** *(2006.01)*

(21) Application number: **10015838.5**

(22) Date of filing: **27.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(62) Document number(s) of the earlier application(s) in accordance with Art. 76 EPC:
**08744448.5 / 2 265 937**

(71) Applicant: **Analogic Corporation**
**Peabody, MA 01960 (US)**

(72) Inventors:
• **Ying, Zhengrong**
**Belmont, MA 02478 (US)**
• **Abenaim, Daniel**
**Lynnfield, MA 01940 (US)**

(74) Representative: **Müller-Boré & Partner**
**Patentanwälte**
**Grafinger Straße 2**
**81671 München (DE)**

(54) **Method of and system for three-dimensional workstation for security and medical applications**

(57) The present invention relates to a system and method for imaging at least a part of an object derived from volumetric data acquired from a scan of at least a portion of the object, comprising: a scanner for acquiring the volumetric data including at least one region of interest; at least two workstations, one configured to generate data displayed on a 2D display device including the region of interest for providing at least one image for initial analysis, and the second configured so as to generate data displayed on a 3D display device including the region of interest for providing at least one image with depth cue for further on-screen analysis if the first workstation can not provide adequate analysis within a predetermined time period. Furthermore, the invention relates to a system for screening luggage comprising: a CT scanner to generate volumetric image data of luggage to be screened; a 2D display workstation for an operator to visualize said volumetric image data to perform visual analysis of scanned luggage; and a 3D display workstation for another operator to visualize said volumetric image data to perform visual analysis of scanned luggage only when said operator can not resolve the scanned luggage using said 2D display workstation within a predetermined time period.

FIG. 6

EP 2 309 257 A1

**Description**

RELATED APPLICATIONS

This patent application and/or patents are related to the following copending U.S. applications and/or issued U.S. patents, of the assignee as the present application, the contents of which are incorporated herein in their entirety by reference:

"Nutating Slice CT Image Reconstruction Apparatus and Method," invented by Gregory L. Larson, et al., U.S. application Ser. No. 08/831,558, filed on Apr. 9, 1997, now U.S. Pat. No. 5,802,134, issued on Sep. 1, 1998;

"Computed Tomography Scanner Drive System and Bearing," invented by Andrew P. Tybinkowski, et al., U.S. application Ser. No. 08/948,930, filed on Oct. 10, 1997, now U.S. Pat. No. 5,982,844, issued on Nov. 9, 1999;

"Air Calibration Scan for Computed Tomography Scanner with Obstructing Objects," invented by David A. Schafer, et al., U.S. application Ser. No. 08/948,937, filed on Oct. 10, 1997, now U.S. Pat. No. 5,949,842, issued on Sep. 7, 1999;

"Computed Tomography Scanning Apparatus and Method With Temperature Compensation for Dark Current Offsets," invented by Christopher C. Ruth, et al., U.S. application Ser. No. 08/948,928, filed on Oct. 10, 1997, now U.S. Pat. No. 5,970,113, issued on Oct. 19, 1999;

"Computed Tomography Scanning Target Detection Using Non-Parallel Slices," invented by Christopher C. Ruth, et al., U.S. application Ser. No. 08/948,491, filed on Oct. 10, 1997, now U.S. Pat. No. 5,909,477, issued on Jun. 1, 1999;

"Computed Tomography Scanning Target Detection Using Target Surface Normals," invented by Christopher C. Ruth, et al., U.S. application Ser. No. 08/948,929, filed on Oct. 10, 1997, now U.S. Pat. No. 5,901,198, issued on May 4, 1999;

"Parallel Processing Architecture for Computed Tomography Scanning System Using Non-Parallel Slices," invented by Christopher C. Ruth, et al., U.S. application Ser. No. 08/948,697, filed on Oct. 10, 1997, U.S. Pat. No. 5,887,047, issued on Mar. 23, 1999;

"Computed Tomography Scanning Apparatus and Method For Generating Parallel Projections Using Non-Parallel Slice Data," invented by Christopher C. Ruth, et al., U.S. application Ser. No. 08/948,492, filed on Oct. 10, 1997, now U.S. Pat. No. 5,881,122, issued on Mar. 9, 1999;

"Computed Tomography Scanning Apparatus and Method Using Adaptive Reconstruction Window," invented by Bernard M. Gordon, et al., U.S. application Ser. No. 08/949,127, filed on Oct. 10, 1997, now U.S. Pat. No. 6,256,404, issued on July 3, 2001;

"Area Detector Array for Computed Tomography Scanning System," invented by David A Schafer, et al., U.S. application Ser. No. 08/948,450, filed on Oct. 10, 1997, now U.S. Pat. No. 6,091,795, issued on July 18, 2000;

"Closed Loop Air Conditioning System for a Computed Tomography Scanner," invented by Eric Bailey, et al., U.S. application Ser. No. 08/948,692, filed on Oct. 10, 1997, now U.S. Pat. No. 5,982,843, issued on Nov. 9, 1999;

"Measurement and Control System for Controlling System Functions as a Function of Rotational Parameters of a Rotating Device," invented by Geoffrey A. Legg, et al., U.S. application Ser. No. 08/948,493, filed on Oct. 10, 1997, now U.S. Pat. No. 5,932,874, issued on August 3, 1999;

"Rotary Energy Shield for Computed Tomography Scanner," invented by Andrew P. Tybinkowski, et al., U.S. application Ser. No. 08/948,698, filed on Oct. 10, 1997, now U.S. Pat. No. 5,937,028, issued on Aug. 10, 1999;

"Apparatus and Method for Detecting Sheet Objects in Computed Tomography Data," invented by Muzaffer Hiraoglu, et al., U.S. application Ser. No. 09/022,189, filed on Feb. 11, 1998, now U.S. Pat. No. 6,111,974, issued on Aug. 29, 2000;

"Apparatus and Method for Eroding Objects in Computed Tomography Data," invented by Sergey Simanovsky, et al., U.S. application Ser. No. 09/021,781, filed on Feb. 11, 1998, now U.S. Pat. No. 6,075,871, issued on Jun. 13, 2000;

"Apparatus and Method for Combining Related Objects in Computed Tomography Data," invented by Ibrahim M. Bechwati, et al., U.S. application Ser. No. 09/022,060, filed on Feb. 11, 1998, now U.S. Pat. No. 6,128,365, issued on Oct. 3, 2000;

"Apparatus and Method for Detecting Sheet Objects in Computed Tomography Data," invented by Sergey Simanovsky, et al., U.S. application Ser. No. 09/022,165, filed on Feb. 11, 1998, now U.S. Pat. No. 6,025,143, issued on Feb. 15, 2000;

"Apparatus and Method for Classifying Objects in Computed Tomography Data Using Density Dependent Mass Thresholds," invented by Ibrahim M. Bechwati, et al., U.S. application Ser. No. 09/021,782, filed on Feb. 11, 1998, now U.S. Pat. No. 6,076,400, issued on Jun. 20, 2000;

"Apparatus and Method for Correcting Object Density in Computed Tomography Data," invented by Ibrahim M. Bechwati, et al., U.S. application Ser. No. 09/022,354, filed on Feb. 11, 1998, now U.S. Pat. No. 6,108,396, issued on Aug. 22, 2000;

"Apparatus and Method for Density Discrimination of Objects in Computed Tomography Data Using Multiple Density Ranges," invented by Sergey Simanovsky, et al., U.S. application Ser. No. 09/021,889, filed on Feb. 11, 1998, now U.S. Pat. No. 6,078,642, issued on Jun. 20, 2000;

"Apparatus and Method for Detection of Liquids in Computed Tomography Data," invented by Muzaffer Hiraoglu, et al., U.S. application Ser. No. 09/022,064, filed on Feb. 11, 1998, now U.S. Pat. No. 6,026,171, issued on Feb. 15, 2000;

"Apparatus and Method for Optimizing Detection of Objects in Computed Tomography Data," invented by Muzaffer Hiraoglu, et al., U.S. application Ser. No. 09/022,062, filed on Feb. 11, 1998, now U.S. Pat. No. 6,272,230, issued on August 7, 2001;

"Multiple-Stage Apparatus and Method for Detecting Objects in Computed Tomography Data," invented by Muzaffer Hiraoglu, et al., U.S. application Ser. No. 09/022,164, filed on Feb. 11, 1998, now U.S. Pat. No. 6,035,014, issued on Mar. 7, 2000;

"Apparatus and Method for Detecting Objects in Computed Tomography Data Using Erosion and Dilation of Objects," invented by Sergey Simanovsky, et al., U.S. application Ser. No. 09/022,204, filed on Feb. 11, 1998, now U.S. Pat. No. 6,067,366, issued on May 23, 2000;

"Apparatus and Method for Classifying Objects in Computed Tomography Data Using Density Dependent Mass Thresholds," invented by Ibrahim M. Bechwati, et al., U.S. application Ser. No. 09/021,782, filed on Feb. 11, 1998, now U.S. Pat. No. 6,076,400, issued on Jun. 20, 2000;

"Apparatus and Method for Detecting Concealed Objects in Computed Tomography Data," invented by Sergey Simanovsky, et al., U.S. application Ser. No. 09/228,380, filed on Jan. 12, 1999, now U.S. Pat. No. 6,195,444, issued on Feb. 27, 2001;

"Apparatus and Method for Optimizing Detection of Objects in Computed Tomography Data," invented by Muzaffer Hiraoglu, et al., U.S. application Ser. No. 09/022,062, filed on Feb. 11, 1998, now U.S. Pat. No. 6,272,230, issued on Aug. 7, 2001;

"Computed Tomography Apparatus and Method for Classifying Objects," invented by Sergey Simanovsky, et al., U.S. application Ser. No. 09/022,059, filed on Feb. 11, 1998, now U.S. Pat. No. 6,317,509, issued on Nov. 23, 2001;

"Apparatus and Method For Processing Object Data in Computed Tomography Data using Object Projections," invented by Carl R. Crawford , et al., U.S. application Ser. No. 09/228379, filed on January 12, 1999, now U.S. Pat. No. 6,345,113, issued on February 5, 2002;

"Apparatus and Method for Detecting Concealed Objects in Computed Tomography Data," invented by Sergey Simanovsky, et al., U.S. application Ser. No. 09/ 228,380, filed on January 12, 1999, now U.S. Pat. No. 6,195,444, issued on February 27, 2001;

"Method of and System for Correcting Scatter in A Computed Tomography Scanner," invented by Ibrahim M. Bechwati , et al., U.S. application Ser. No. 10/121,466, filed on April 11, 2002, now U.S. Pat. No. 6,687,326, issued on February 3, 2004;

"Method of and System for Reducing Metal Artifacts in Images Generated by X-Ray Scanning Devices," invented by Ram Naidu, et al., U.S. application Ser. No. 10/171,116, filed on June 13, 2002, now U.S. Pat. No. 6,721,387, issued on April 13, 2004;

"Method and Apparatus for Stabilizing the Measurement of CT Numbers," invented by John M. Dobbs, U.S. application Ser. No. 09/982,192, filed on October 18, 2001, now U.S. Pat. No. 6,748,043, issued on June 8, 2004;

"Method and Apparatus for Automatic Image Quality Assessment," invented by Seemeen Karimi, et al., U.S. application Ser. No. 09/842,075, filed on April 25, 2001, now U.S. Pat. No. 6,813,374, issued on November 2, 2004;

"Decomposition of Multi-Energy Scan Projections using Multi-Step Fitting," invented by Ram Naidu, et al., U.S. application Ser. No. 10 / 611,572, filed on July 1, 2003, now U.S. Pat. No. 7,197,172, issued on March 27, 2007;

"Method of and System for Detecting Threat Objects using Computed Tomography Images," invented by Zhengrong Ying, et al., U.S. application Ser. No. 10/831,909, filed on April 26, 2004, now U.S. Pat. No. 7,277,577, issued on October 2, 2007;

"Method of and System for Computing Effective Atomic Number Image in Multi-Energy Computed Tomography," invented by Zhengrong Ying, et al., U.S. application Ser. No. 10/850,910, filed on May 21, 2004, now U.S. No. 7,190,757, issued on March 13, 2007;

"Method of and System for Adaptive Scatter Correction in Multi-Energy Computed Tomography," invented by Zhengrong Ying, et al., U.S. application Ser. No. 10/853,942, filed on May 26, 2004, now U.S. Pat. No. 7,136,450, issued on Nov. 14, 2006;

"Method of and System for Destreaking the Photoelectric Image in Multi-Energy Computed Tomography," invented by Zhengrong Ying, et al., U.S. application Ser. No. 10/860,984, filed on June 4, 2004 (Attorney's Docket No. 56230-609 (ANA-256));

"Method of and System for Extracting 3D Bag Images from Continuously Reconstructed 2D Image Slices in Computed Tomography," invented by Zhengrong Ying, et al., U.S. application Ser. No. 10/864,619 , filed on June 9, 2004, now U.S. Pat. No. 7,327,853, issued on February 5, 2008;

"Method of and System for Sharp Object Detection using Computed Tomography Images," invented by Gregory L. Larson, et al., U.S. application Ser. No. 10/883,199, filed on July 1, 2004, now U.S. Pat. No. 7,302,083, issued on November 27, 2007;

"Method of and System for X-Ray Spectral Correction in Multi-Energy Computed Tomography," invented by Ram Naidu, et al., U.S. application Ser. No. 10/899,775, filed on July 17, 2004, now U.S. Pat. No. 7,224,763, issued on May 29, 2007;

"Method of and System for Detecting Anomalies in Projection Images Generated by Computed Tomography Scanners," invented by Anton Deykoon, et al., U.S. application Ser. No. 10/920,635, filed on Aug. 18, 2004 (Attorney's Docket No. 56230-614 (ANA-260));

"Method of and System for Stabilizing High Voltage Power Supply Voltages in Multi-Energy Computed Tomography," invented by Ram Naidu, et al., U.S. application Ser. No. 10/958,713, filed on Oct. 5, 2004, now U.S. Pat. No. 7,136,451, issued on Nov. 14, 2006;

"Method of and System for 3D Display of Multi-Energy Computed Tomography Images," invented by Zhengrong Ying, et al., U.S. application Ser. No. 11/142,216, filed on June 1,2005 (Attorney's Docket No. 56230-625 (ANA-267));

"Method of and System for Classifying Objects using Local Distributions of Multi-Energy Computed Tomography Images," invented by Zhengrong Ying, et al., U.S. application Ser. No. 11/183,471, filed on July 18, 2005 (Attorney's Docket No. 56230-626 (ANA-268));

"Method of and System for Splitting Compound Objects in Multi-Energy Computed Tomography Images," invented by Sergey Simanovsky, et al., U.S. application Ser. No. 11/183,378, filed on July 18, 2005 (Attorney's Docket No. 56230-627 (ANA-269));

"Method of and System for Classifying Objects using Histogram Segment Features in Multi-Energy Computed Tomography Images," invented by Ram Naidu, et al., U.S. application Ser. No. 11/198,360, filed on Aug. 4, 2005 (Attorney's Docket No. 56230-628 (ANA-270));

"Method of and System for Automatic Object Display of Volumetric Computed Tomography Images for Fast On-Screen Threat Resolution," invented by Zhengrong Ying, et al., U.S. application Ser. No. 11/704,482, filed on February 9, 2007 (Attorney's Docket No. 56230-638 (ANA-279)); and

"Method of and System for Variable Pitch Computed Tomography Scanning for Baggage Screening," invented by Ram Naidu, et al., U.S. application Ser. No. 11/769,370, filed on June 27, 2007 (Attorney's Docket No. 56230-641 (ANA-281)).

FIELD OF THE DISCLOSURE

The present disclosure relates to methods of and systems for processing volumetric data generated by scanners, such as CT scanners, MRI scanners, ultrasound scanners, and tomosynthesis scanners; and more particularly to a method of and a system for displaying objects of volumetric data onto a 2D or 3D display with applications to surgical preparation and planning in medical domains, baggage and parcel screening in security areas; and any other type of scanning.

BACKGROUND OF THE DISCLOSURE

Various types of scanning systems are known for creating volumetric image data for display, including those in the medical and security fields. Medical scanners, such as CT, MRI, ultrasound and tomosynthesis scanners are essential diagnostic tools of the medical professionals for scanning internal parts of a body, while security CT scanners are used to detect the presence of explosives and other prohibited items prior to loading the baggage and parcels onto a commercial aircraft.

In a typical medical application, a radiologist uses a 2D display for diagnostic purposes by looking at the images rendered from the volumetric image data acquired from a scanner to determine if a patient has a particular disease. In certain security applications, automatic threat detection methods are used to detect potential threats. Such methods can yield a certain percentage of false alarms usually requiring operators to intervene to resolve any falsely detected bags. It is very labor intensive to open a bag and perform a hand-search each time. Therefore, it is desirable to display the volumetric image data in combination with the automatic threat detection results on a 3D display device, such as the "Volumetric three-dimensional display system" invented by Dorval, et al. (U.S. Pat. No. 6,554,430), or on a 2D LCD/CRT display with 3D volume rendering using techniques such as the "Volume rendering techniques for general purpose graphics hardware" by Christof Rezk-Salama in his Ph. D. dissertation at University of Erlangen in December 2001.

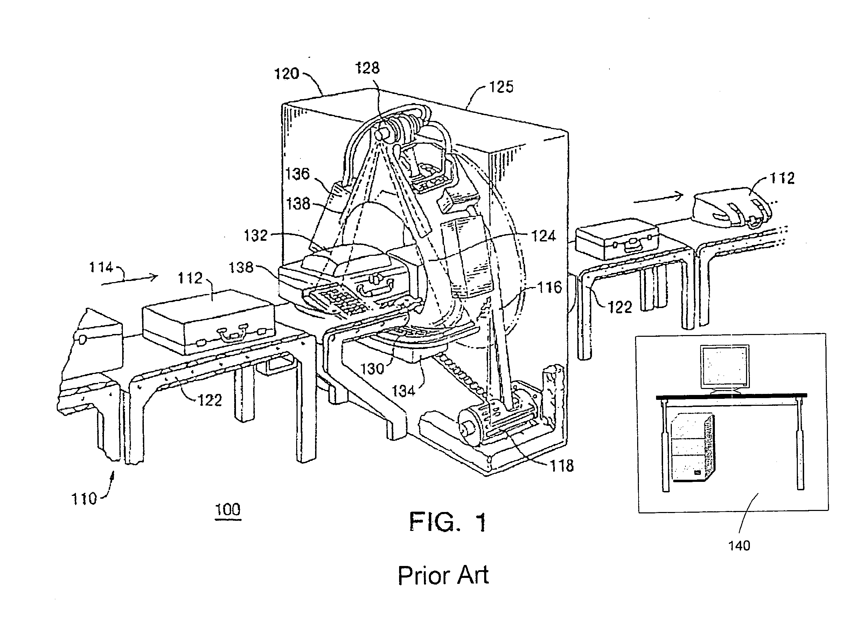

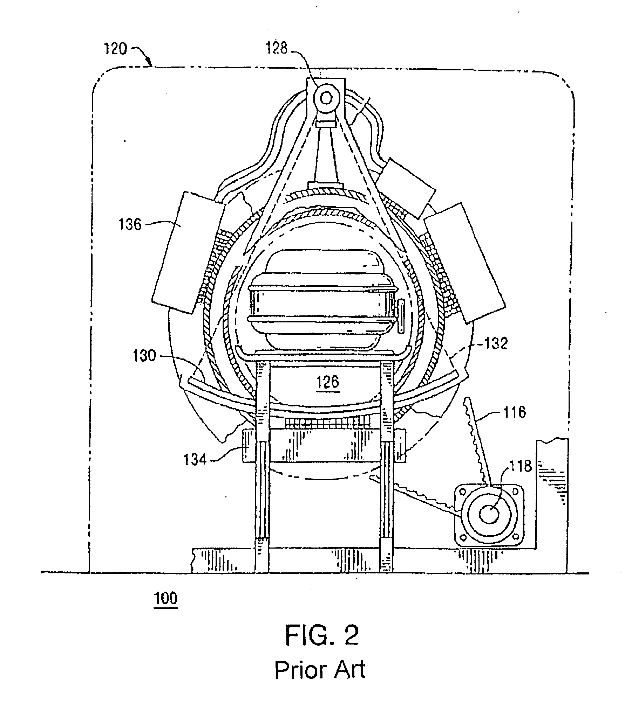

Referring to the drawings, FIGs. 1,2 and 3 show perspective, end cross-sectional and radial cross-sectional views, respectively, of a typical baggage scanning system 100 simplified for exposition. System 100 includes a conveyor system 110 for continuously conveying baggage or luggage 112 in a direction indicated by arrow 114 through a central aperture of a CT scanning system 120. The conveyor system includes motor driven belts for supporting the baggage. Conveyer system 110 is illustrated as including a plurality of individual conveyor sections 122; however, other forms of conveyor systems may be used.

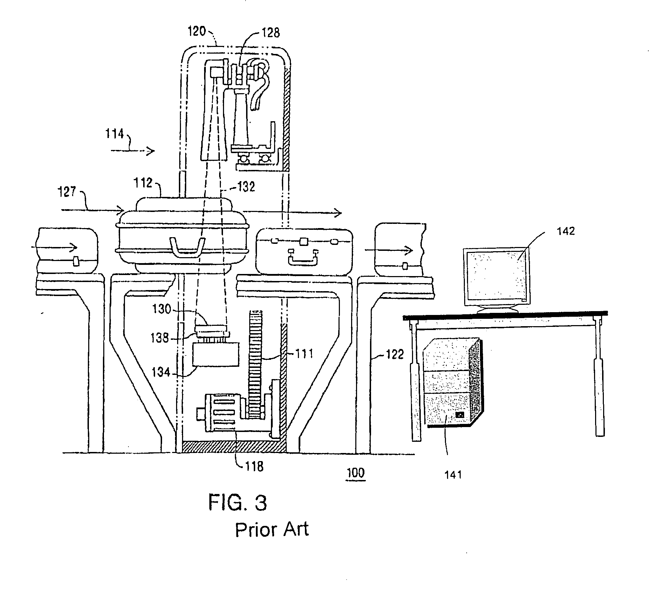

The CT scanning system 120 includes an annular shaped rotating platform, or disk, 124 disposed within a gantry support 125 for rotation about a rotation axis 127 (shown in FIG. 3) that is preferably parallel to the direction of travel 114 of the baggage 112. Disk 124 is driven about rotation axis 127 by any suitable drive mechanism, such as a belt 116 and motor drive system 118, or other suitable drive mechanism. Rotating platform 124 defines a central aperture 126 through which conveyor system 110 transports the baggage 112.

The system 120 includes an X-ray tube 128 and a detector array 130 which are disposed on diametrically opposite sides of the platform 124. The detector array 130 is preferably a two-dimensional array. The system 120 further includes a data acquisition system (DAS) 134 for receiving and processing signals generated by detector array 130, and an X-ray tube control system 136 for supplying power to, and otherwise controlling the operation of, X-ray tube 128. The system 120 is also preferably provided with a computerized system 140 for processing the output of the data acquisition system 134 and for generating the necessary signals for operating and controlling the system 120. The computerized system can also include a monitor 142 for displaying information including generated images. System 120 also can include shields 138, which may be fabricated from lead, for example, for preventing radiation from propagating beyond gantry 125. Alternatively, the entire CT scanning system can be disposed within an enclosed housing (not shown) containing lead, with a suitable entry and exit for the items on the conveyor system 110, in order to provide proper shielding to protect personnel in the vicinity of the scanner from stray radiation.

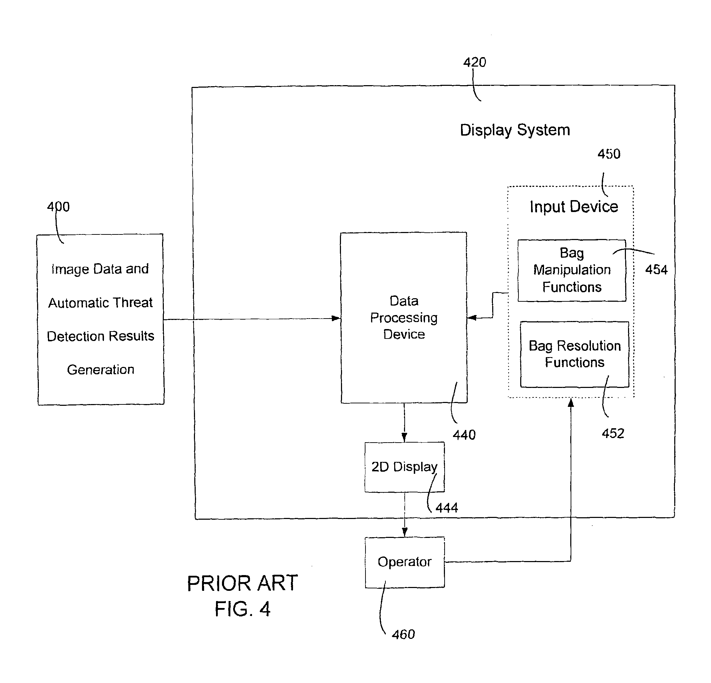

FIG. 4 shows an example of a prior art display system for on-screen threat resolution. Volumetric image data including CT images and Z (atomic number) images, and automatic threat detection results are generated in unit 400, and are fed into the display system 420. The display data processing device 440 processes the CT images, Z images, and the automatic threat detection results to generate display images for the 2D display device 444. An operator 460 looks at the 2D display device 444 and uses the input device 450 to interact with the 2D display system. The input device can be divided into two groups of functions: bag resolution functions 452 and bag image manipulation functions 454. The bag resolution functions 452 include one or more recommended actions for each detected threat object in the bag as well as one or more recommended actions for the bag. The recommended actions for threat objects can include clearing an object, suspecting an object for further investigation, or alarming an object. The recommended actions for a bag can also include clearing the bag, suspecting the bag, or alarming the bag based on the aggregate actions for all the detected potential threat objects. The bag image manipulation functions 454 may include different functions for manipulating the image of the bag, such as rotating the 2D image plane through the bag, etc.

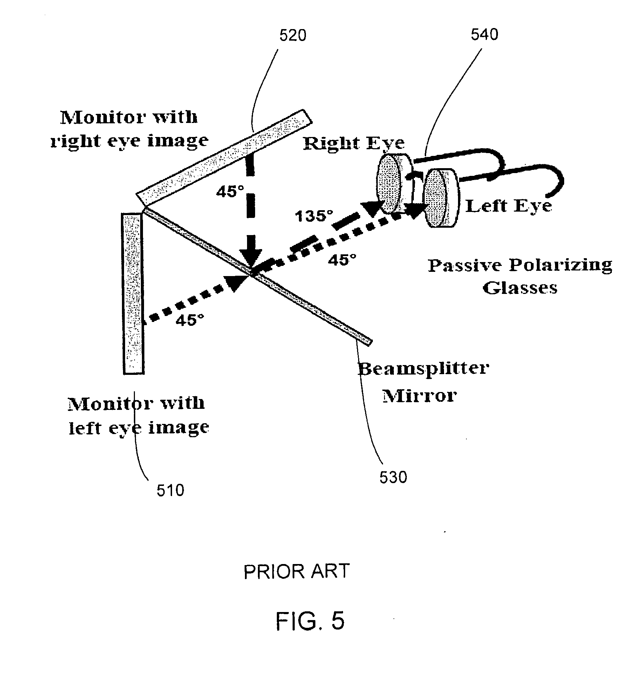

Three-dimensional (3D) displays have been developed mostly for gaming purposes. These three-dimensional displays include volumetric displays, stereoscopic displays, and holographic displays. FIG. 5 illustrates one type of 3D stereoscopic display described in Fergason, et al., "An innovative beamsplitter-based stereoscopic 3-D display design," Proc SPIE 5664, 488 (2005) (hereinafter referred to as "FERGASON's 3D DISPLAY"). A viewer wearing passive polarizing glasses 540 is able to see a 3D effect, i.e. the depth information of a 3D scene or a 3D object. The left eye of the viewer receives the left eye image transmitted through the beamsplitter mirror 530 from the monitor with left eye image 510; the right eye of the viewer receives the right eye image reflected by the beamsplitter mirror 530 from the monitor with right eye image 520. When the left eye image and right eye image have an appropriate disparity of a three-dimensional scene or rendered volume data, the viewer sees the depth of the scene or the volume data. Note that the polarization takes place on both monitors with the monitor 510 matching the left eye glass and with the monitor 520 matching the right eye glass. However, directly connecting the data processing device 440 of prior art system shown in FIG. 4 to a three-dimensional display does not generate any 3D effect to a viewer, but might generate many image artifacts that make viewers uncomfortable.

In the past, displayed images will include highlighting achieved by coloring the whole area of the object with a different color such as red. The human eyes are less sensitive to a color than to grayscale, thus coloring the whole object causes human eyes to lose the ability to discern the detail structure of the object.

SUMMARY OF THE DISCLOSURE

In accordance with one aspect of the present disclosure, a method of rendering volumetric data includes highlighting a detected object using the contour of the object onto a 2D display. The volumetric data can be generated by any type of imaging system, including medical and baggage scanners. The method comprises two real-time rendering passes: one pass for rendering the volumetric data without highlighting a detected object; the other pass for rendering only the detected object to generate a 2D binary projection image. The rendering passes can both take place inside a graphics processing unit (GPU) for speed and efficiency. The 2D binary projection image is then processed to extract a contour of the detected object using, for example, an edge detection filter. The extracted contour is then colored differently from the image rendered in the first pass. The colored contour and the image rendered in the first pass are composited into a final display image, which is shown on a 2D display for visualization. The method of the present disclosure improves the readability of displayed grayscale image data of a part of an object derived from the volumetric data acquired from a scan of at least a portion of the object by processing the volumetric data to identify at least one region of interest in the object and highlighting the boundary that defines each region of interest with a color using for example the GPU, while preserving the gray-scaled details within each region of interest.

In accordance with another aspect of the present disclosure, a method of real-time rendering volumetric data comprises highlighting a detected object with the contour of the object being extracted by using, for example a GPU, and displaying the rendered images onto a 3D stereoscopic display. In accordance with one embodiment, the method comprises generating contour data representing voxels from a 3D contour volume corresponding to a detected object; generating RGBA volume data from indexed volume data with a look-up-table of one or more desired colors and opacities for visualization; replacing the voxels in the RGBA volumetric data corresponding to the voxels in the 3D contour volume with a pre-selected color for highlighting; rendering the contour highlighted RGBA volume into a left eye image and a right eye image and display the left eye image and the right eye image for the 3D stereoscopic display.

In accordance with one aspect of the present disclosure, a scanner can be used to scan potential threat objects and generate volumetric image data corresponding to the scanned objects, and a threat detection system can be configured to include a list of pre-selected types for threat detection. The threat detection system can generate label image data, which defines each potential threat object as a separate region of interest.

In accordance with yet another aspect of the present disclosure, a 3D workstation using a 3D stereoscopic display is provided for real-time visualization. The 3D workstation comprises a graphic processing unit (GPU), which implements the contour highlighting algorithms for visualization. The 3D workstation is configured to process single energy CT data, dual or multi-energy CT data, MRI data, tomosynthesis scanning data, and 3D ultra-sound data. The applications of the workstation include both security luggage screening and medical domains such as surgical preparation, guided surgery, surgery explanation to patients, and diagnosis.

In accordance with still another aspect of the present disclosure, a system for screening checked luggage or/and carry-on luggage with detection of predetermined types of threat objects is also provided. The system comprises a CT scanner, a threat detection system, a 2D workstation, and a 3D workstation. The 3D workstation is used in conjunction of the 2D workstation to perform further on-screen analysis of complex luggage when the 2D workstation can not resolve the scanned luggage within a predetermined time period. The 3D workstation can also be used to assist operators to open and perform a hand search of a suspected bag.

BRIEF DESCRIPTION OF THE DRAWINGS

The drawing figures depict preferred embodiments by way of example, not by way of limitations. In the figures, like reference numerals refer to the same or similar elements.

FIG. 1 is a perspective view of a baggage scanning system, known in the prior art, and which can be adapted to incorporate the systems and perform the methods described herein.

FIG. 2 is a cross-sectional end view of the system of FIG. 1.

FIG. 3 is a cross-sectional radial view of the system of FIG. 1.

FIG. 4 is a flow block diagram illustrating the logical flow of a prior art display system for on-screen threat resolution.

FIG. 5 is an illustration of a prior art 3D stereoscopic display.

FIG. 6 is a flow block diagram illustrating the logical flow of one embodiment of a system configured to visualize 3D volumetric CT images with automatic threat detection results on a 3D stereoscopic display of the present disclosure.

FIG. 7 is a block diagram illustrating the logical flow of one embodiment of highlighting an object using a contour of the object on a 2D display of the present disclosure.

FIG. 8 is a block diagram illustrating the logical flow of one embodiment of highlighting an object using a contour of the object on a 3D stereoscopic display of the present disclosure.

FIG. 9 is a block diagram illustrating the logical flow of one embodiment of a security luggage screening system using a 3D display workstation of the present disclosure.

FIG. 10 is a block diagram illustrating the logical flow of one embodiment of a security luggage screening system using a 3D display workstation of the present disclosure.

DETAILED DESCRIPTION OF THE PREFERRED EMBODIMENTS

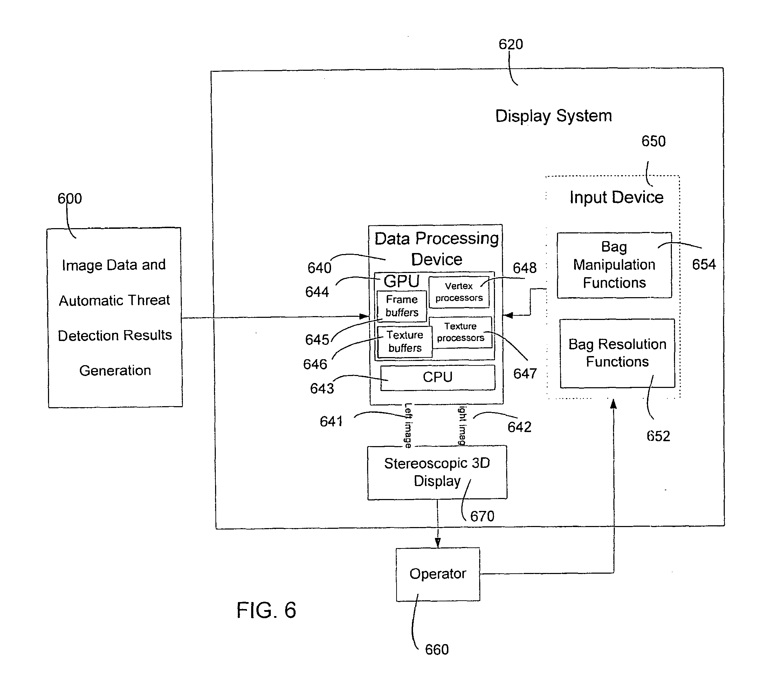

FIG. 6 shows a flow diagram illustrating one embodiment of the logical flow of one embodiment of visualizing 3D volumetric CT images with automatic threat detection results on a 3D stereoscopic display device, such as a FERGASON's 3D DISPLAY, for fast on-screen threat resolution. In this embodiment the volumetric CT image data is generated by a CT scanner, and comprises data representing a plurality of voxels representing a scanned object, each of which has a numerical value assigned to it which represents a density measurement of the represented voxel. The density measurement is determined as a function of the measure of X-ray attenuation through the mass of the object represented by the voxel during a scan. For example, water has a physical density of 1 g/cc, and a voxel representing the CT image density measurement of water has a numerical value of 1000 Hounsfield Units (HU). In the case of dual energy CT scanning, the volumetric CT image data also includes volumetric effective atomic number (Z) image data. The effective atomic number (Z) image data also comprises a plurality of voxels, each of which represents an effective atomic number measurement of scanned objects; for example, Aluminum has an atomic number of 13, and the Z image of Aluminum has value of 1300 Z Units (ZU).

Still referring to FIG. 6, the volumetric CT image data are fed into an automatic threat detection system, generally referenced at 600, which in turn generates data containing detection results for the display system 620 in accordance with the present disclosure. In one embodiment the automatic threat detection unit uses one or more of the methods described in the assignee's "Apparatus and method for eroding objects in computed tomography data," invented by Sergey Simanovsky, et al., U.S. Pat. No. 6,075,871, issued on Jun. 13, 2000, incorporated herein by reference; "Apparatus and method for combining related objects in computed tomography data," invented by Ibrahim M. Bechwati, et al., U.S. Pat. No. 6,128,365, issued on Oct. 3, 2000, incorporated herein by reference; "Apparatus and method for detecting sheet objects in computed tomography data," invented by Sergey Simanovsky, et al., U.S. Pat. No. 6,025,143, issued on Feb. 15, 2000, incorporated herein by reference; "Apparatus and method for classifying objects in computed tomography data using density dependent mass thresholds," invented by Ibrahim M. Bechwati, et al., U.S. Pat. No. 6,076,400, issued on Jun. 20, 2000, incorporated herein by reference.

In one embodiment, the automatic threat detection system generates label image volumetric data, in which all the voxels of a detected threat are assigned a same unique positive integer number. For example, if there are three detected threats in a bag, the corresponding label image data will have labels from one to three respectively indicating the first, second, and third threat objects; the voxels of the first object are all assigned with a label value of one in the label image data, and so on; the voxels that do not belong to any threat object are assigned a label value of zero.

As shown in FIG. 6, the illustrated embodiment of the data processing device 640, which includes a graphics processing unit (GPU) 644, receives volumetric image data and label image data and generates two display images, left eye image 641 and right eye image 642, rendered from the volumetric CT data and the label data using the methods described in the assignee's "Method of and System for 3D Display of Multi-Energy Computed Tomography Images," invented by Zhengrong Ying, et al., U.S. application Ser. No. 11/142,216, filed on June 1. 2005 (Attorney's Docket No. 56230-625 (ANA-267)) (hereinafter referred to as "Assignee's 3D RENDERING application"); "Method of and System for Automatic Object Display of Volumetric Computed Tomography Images for Fast On-Screen Threat Resolution," invented by Zhengrong Ying, et al., U.S. application Ser. No. 11/704,482, filed on February 9, 2007 (Attorney's Docket No. 56230-638 (ANA-279)) (hereinafter referred to as "Assignee's AOD application"); all incorporated herein in their entirety by reference. The two display images 641 and 642 are generated with a disparity angle usually, although not necessarily, ranging from 1.5 degrees to 10 degrees. The disparity angle can be set in a configuration file or can be adjusted from the input device 650. The two display images 641 and 642 may then be displayed on a 3D display device 670 such as FERGASON's 3D DISPLAY. An operator 660 looks at the 3D display device 670 and uses the input device 650 to interact with the 3D display system. The data processing device 640 updates the left eye and right eye images according to the requests collected from the user input device 650 so that volumetric data is displayed according to a user's desire. Since the operator has the capability of seeing the scanned objects or luggage in 3D to better understand the spatial relationship of objects, the operator can reduce the time in using bag manipulation functions 654 and increase the accuracy in resolving alarmed threat objects through the bag resolution functions 652.

In one embodiment of the present disclosure, a method of highlighting objects using contours or boundaries for a 2D display is described in detail. In another embodiment of the present disclosure, a contour highlighting algorithm for a 3D stereoscopic display is also described. Object highlighting using contours in accordance with the present disclosure, can attract an operator's attention, while preserving the detailed structure inside the object in grayscale. Further, while object highlighting using contours is described herein as very using with gray scale images, such contour highlighting can be used with any type of image representing density measurements. For example, where pseudo-color schemes are used to represent different density measurements within an image, the color contouring of an object will make it clear which objects are of interest.

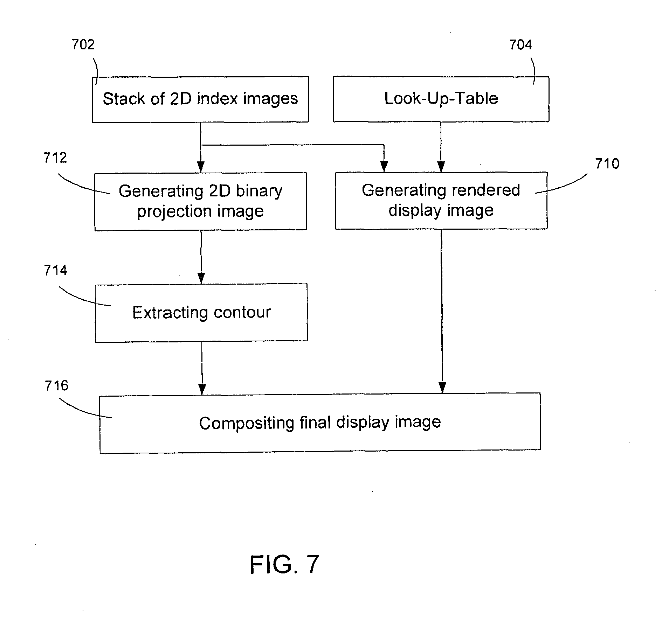

FIG. 7 shows a block diagram of one embodiment illustrating the logical flow of highlighting an object using a contour of the object on a 2D display. A stack of 2D index images 702 contains the information associated with 3D volumetric CT image data and label image data. A look-up-table 704, which is generated according to the desirable colors and opacity by a user, is stored in a Graphics Processor Unit (GPU) 644 as shown in FIG. 6. In one embodiment, the stack of 2D index images is generated from the volumetric CT image data and label image volumetric data according to the methods described in the Assignee's 3D REDERING and AOD applications. The stack of 2D index image can either be generated according to the original sampling grids of the CT volumetric data or can be generated by re-sampling the CT volume in a desirable way. The stack of the 2D index images and the look-up-table are rendered and processed in texture processors 646 as shown in FIG. 6 in Step 710 by using, for example, the methods described in Assignee's 3D RENDERING and AOD applications into a 2D display image stored, for example, in a texture buffer 646 instead of a frame buffer 645 as shown in FIG. 6.

Referring to Step 712 of FIG. 7, a 2D binary projection image corresponding to a selected object to be highlighted is also generated from the stack of the 2D index images. Given the orientation parameters at which a volume is desired to be displayed, one embodiment for generating a 2D binary projection image corresponding to a selected object is to store the relevant data in a texture buffer of a GPU. The embodiment further comprises the following steps:

    A. For each 2D index image, generate a binary image by setting the voxels corresponding to the selected object label value to one and setting the rest of the voxels to zero;

    B. Rotate each 2D index image according to the orientation parameters by using the nearest neighbor interpolation scheme;

    C. Set the pixel value of the 2D binary projection image in the texture buffer to one for the pixels on which any non-zero voxels from the binary image are projected; and

D. Set the rest of the pixels of the 2D binary projection image in the texture buffer to zero.

By performing the above steps, a 2D binary projection image corresponding to a selected object is generated and stored in a texture buffer of a GPU.

Referring to Step 714 of FIG. 7, edge detection is performed on the 2D binary projection image to extract the contour of the selected object for highlighting. Any one of several edge detection techniques can be employed. In one embodiment, given the binary projection image with size of *IxJ,* denoted by *P(i, j),* the output binary image containing the extracted contour, denoted by *C(i, j),* where $i = 0, \cdots, I\text{-}1; j = 0, \cdots, J\text{-}1$, is computed using an edge filter as follows,

$$C(i, j) = \prod_{i'=i-1; j'=j-1}^{i'=i+1; j'=j+1} P(i', j')$$

A pixel is detected as an edge pixel when any of its eight neighboring pixels in the three by three square centered by the pixel has a zero-valued pixel, and the pixel is assigned a value of one; otherwise, the pixel is assigned a value of zero denoting a non-edge pixel.

Referring to Step 716 of FIG. 7, the final display image is generated by compositing the rendered image of Step 710 and the extracted contour of Step 714, that is, the contour pixels of the selected object as indicated by the binary contour image are replaced by a pre-chosen highlighting color for the object, and the other pixels from the rendered image remain the same for final display. Note that in this embodiment the final display image is directly generated at the frame buffer 645 of the GPU 644 as shown in FIG. 6.

In another embodiment of the present disclosure, the extracted contour using an edge filter using the 2D binary projection image can be dilated into a thicker edge of the selected object in order to be more visible to an operator. The number of the dilations can be configured or adjusted to the preference of individual operators.

The contour highlighting algorithm described above does not work for the 3D stereoscopic display. Because the contours extracted for the left eye image and right eye image do not originate from the same points in the volumetric data sets, the contours do not form correct disparity for the left eye and right eye, resulting in uncomfortable viewing in the 3D stereoscopic display.

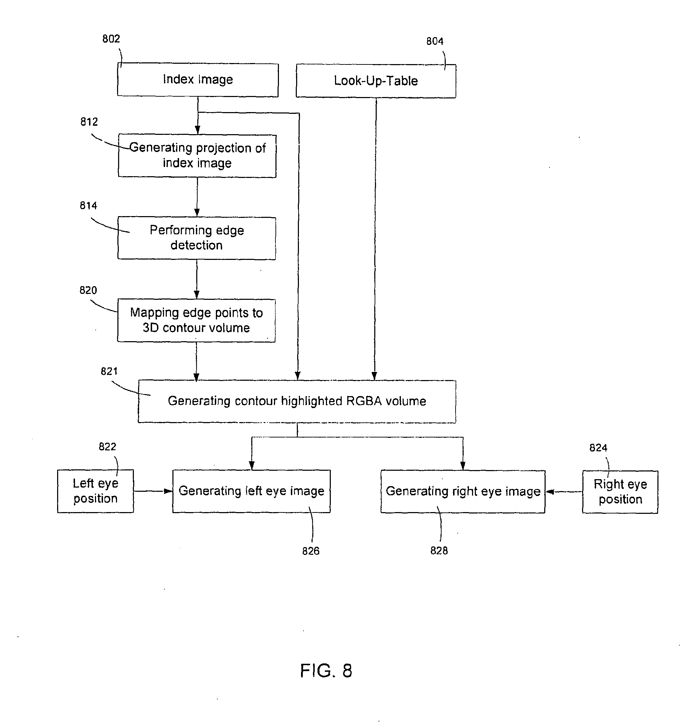

FIG. 8 shows a block diagram which illustrates the logical flow of one embodiment of highlighting an object using a contour of the object on a 3D stereoscopic display for more comfortable viewing. Steps 812 and 814 of FIG. 8 can remain the same as steps 712 and 714 of FIG. 7. In Step 820 of FIG. 8, the extracted contour in the projection image of a selected object is mapped back to the 3D volume of the data. For computational efficiency, the 3D volume of the data has the same size as the stack of the index images. The 3D volume of data containing the contour points of the selected object is herein referred to as the "3D contour volume", and, in one embodiment, is generated using the following steps:

A. First the binary image containing the extracted contour of the selected object is rotated and resized to the same size as each index image by using the nearest neighbor interpolation scheme.

B. Then a 3D contour volume is generated by comparing the rotated resized binary contour image with each index image. The voxels that are the voxels of the selected object in the index image and are the contour pixels of the binary contour image are set to one; and the other voxels in the 3D volume are set to zero.

Referring to FIG. 8, in one embodiment Step 826 uses the 3D contour volume, the stack of 2D index images, the look-up-table, and the left eye position to view the data to generate a left eye image for the 3D stereoscopic display using the following steps:

A. For each index image, perform a table look up to convert the index image into an RGBA image;

B. Replace the pixels in the RGBA image which have values of one in the 3D contour volume with a desired color for contour highlighting of the selected object to generate a contour highlighted RGBA image as shown in Step 821;

C. Rotate the contour highlighted RGBA image according to the left eye position 826 and orientation parameters by interpolation to generate a rotated RGBA image with contour highlighting; and

D. Blend all rotated RGBA images with contour highlighting from back to front according to the opacity values defined in the A channel to generate a left eye image.

In the illustrated embodiment, the right eye image at Step 828 of FIG. 8 is generated with the same steps as above, but at the right eye position 824. After the left eye image and right eye image are generated, they are sent to the left eye monitor and right eye monitor to display. Note that the above described processing steps are implemented in a GPU to obtain the real-time rendering speed so that a user interacting with the images does not feel a delay. Total rendering time of one pair of images less than 50 milliseconds suffices the real-time requirements, although this can vary to some extent. A 3D effect of the displayed volume with contour highlighting of a selected object can be observed, which allows a user to pay attention of the highlighted object but also discern the detail of the object simultaneously.

In one embodiment of the present disclosure, the volumetric data is first converted into a stack of 2D index images, which is also called an index volume. The index volume can be processed as a whole volume instead of one 2D index image at a time. An RGBA volume is generated directly from the index volume. The contour highlighted RGBA volume is generated directly from the RGBA volume and 3D contour volume. The left eye and right images can then be generated from the contour highlighted RGBA volume directly.

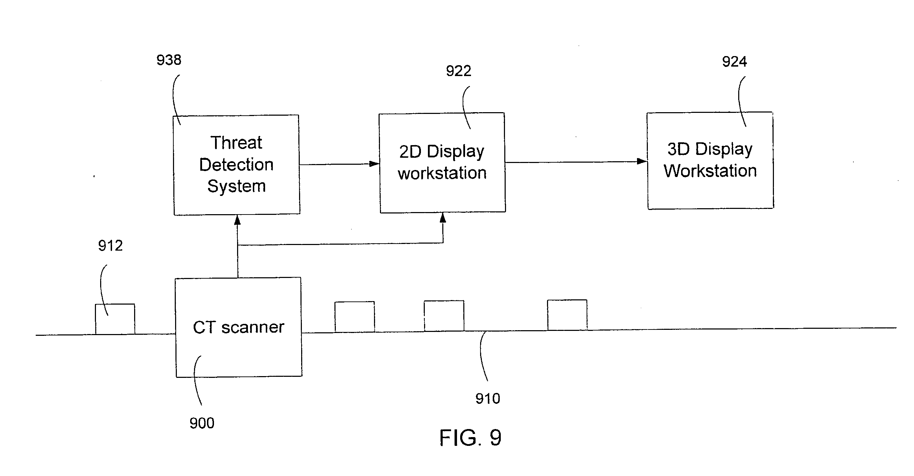

In one embodiment of the present disclosure shown in FIG. 9, a 3D display workstation is used in conjunction with a 2D display workstation for security luggage screening. FIG. 9 shows a logical flow of such a security luggage screening system. The security luggage screening system, for example, can be used for checked luggage screening, carry-on luggage screening at checkpoint, or any entrances or gates of buildings, stadiums, bus stations, or rail way stations. A parcel or a piece of luggage 912 is carried through CT scanner 900 using a conveyer system 910. The volumetric CT image data of the scanned item is sent to a threat detection system 938, which generates label image data containing the results of the threat detection. The volumetric CT image data and label data are sent to a 2D display workstation 922. The volumetric CT image data and label data are rendered to the 2D display. Operators examine the contents of the bag with highlighted threat objects. When operators can not render a decision on a particular object or bag because of insufficient time or the complexity of the bag and/or its contents, the volumetric CT image data and the label data are sent to a 3D display workstation 924. The 3D display workstation generates 3D images for an operator to examine when the operator using 2D workstation can not resolve the scanned item. Because of the depth cue in the 3D display, operators have a better understanding of the contents of a bag, which helps to resolve threat objects on screen, reducing the labor cost and time for hand search a bag. Each workstation comprises a computer and a graphics processing unit for receiving data, storing data, and rendering data to display images. However, it is desirable that the 3D display workstation and 2D display work station employ only one physical computer and one physical GPU for both workstations so as to eliminate the data transfer and communication overhead. One computer and one GPU can be virtually partitioned for simultaneous use with the 2D display workstation and the 3D display workstation.

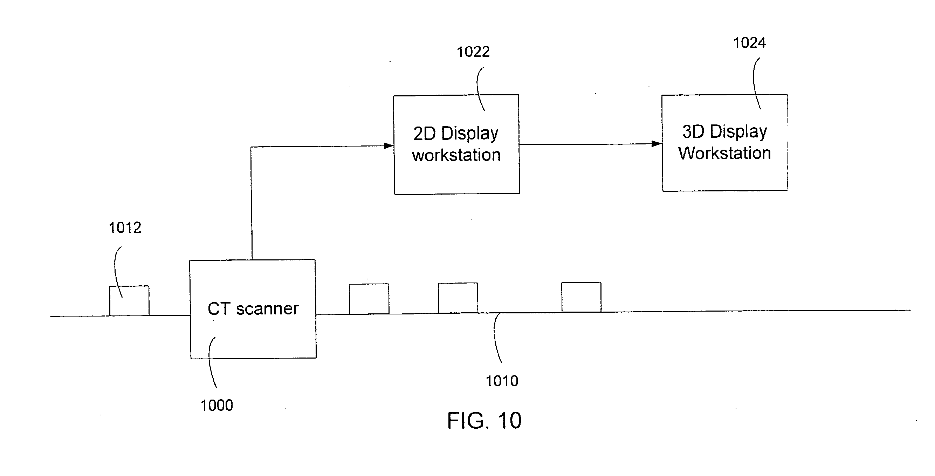

FIG. 10 shows a block diagram illustrating the logical flow of one embodiment of a security screening system using both a 2D display workstation and a 3D display workstation. In this embodiment, the threat detection system is not present so that an operator must use the 2D display workstation 1022 to visually interpret the content of a scanned item. The 3D workstation 1024 is used when the operator using the 2D display workstation can not resolve a scanned item because of insufficient time or the complexity of the scanned item. The 3D workstation can also be used to assist operators to locate objects when searching a bag.

In one embodiment of the present disclosure, the volumetric image data includes volumetric atomic number image data from a dual or multi-energy CT scanner. The index image data and look-up-tables from the volumetric CT image data, volumetric atomic number image data, and label image data of threat detection results can be generated, for example, by using the method described in Assignee's 3D REDERING application.

In another embodiment of the present disclosure, the stack of 2D index images and look-up-tables are generated without using the label image data of the threat detection results. In some applications, for example, carry-on luggage screening using CT scanners may only require visual inspection of the contents of scanned luggage by operators without automatic threat detection.

While this disclosure has been particularly shown and described with references to preferred embodiments thereof, it will be understood by those skilled in the art that various changes in form and details may be made therein without departing from the spirit and scope of the disclosure as defined by the following claims. These variations from the preferred embodiment of the present disclosure include extending the security screening system to medical applications. In these medical applications, patients instead of luggage are scanned by a CT scanner and the reconstructed images are visualized by either 2D display workstation or 3D display workstation, or on both workstations. A radiologist, a surgeon, or other physicians then use the 2D display workstation and the 3D display workstation to diagnose the patient, prepare for a surgery, and/or use the 3D display workstation to guide a surgery. Furthermore, volumetric image data from other modalities such as a 3D ultra-sound scanner, a Magnetic Resonance Imaging (MRI) scanner, or a tomosynthesis scanner can also be rendered and visualized on the 3D display workstation. Other types of 3D display can also be used by converting the 3D volumetric data set into a 3D display set which can be displayed directly on the 3D display. When the 3D volumetric data is time-varying, the 3D display workstation can be used to display the time-varying 3D volumetric data by updating the difference of two consecutive 3D volumetric data sets.

## Claims

1. A system for imaging at least a part of an object derived from volumetric data acquired from a scan of at least a portion of the object, comprising:

a scanner for acquiring the volumetric data including at least one region of interest;
at least two workstations, one configured to generate data displayed on a 2D display device including the region of interest for providing at least one image for initial analysis, and the second configured so as to generate data displayed on a 3D display device including the region of interest for providing at least one image with depth cue for further on-screen analysis if the first workstation can not provide adequate analysis within a predetermined time period.

2. The system according to claim 1, further including a threat detection subsystem.

3. The system according to claim 2, wherein the workstation configured to generate data for displaying an image on each of the display devices is provided data from the threat detection subsystem.

4. The system according to claim 3, wherein the scanner is configured to scan luggage and the threat detection system is configured to detect predetermined types of threat objects which define the regions of interest.

5. The system according to claim 4, wherein the workstation configured so that the workstation generating data displayed on the 3D display device is used by an operator, when the operator is unable to make a determination whether a threat object is present from inspecting the data displayed on the 2D display device.

6. A method of imaging at least a part of an object derived from volumetric data acquired from a scan of at least a portion of the object, comprising:

    acquiring the volumetric data including at least one region of interest;
    using at least two workstations, one configured to generate data displayed on a 2D display device including the region of interest for providing at least one image for initial analysis, and the second configured so as to generate data displayed on a 3D display device including the region of interest for providing at least one image with depth cue for further on-screen analysis if the first workstation can not provide adequate analysis within a predetermined time period.

7. The method according to claim 6, detecting whether the acquired volumetric data includes a threat object.

8. The method according to claim 7, further including providing data to the 2D workstation associated with the detected threat.

9. The method according to claim 8, wherein acquiring the volumetric data includes scanning luggage for predetermined types of threat objects which define the regions of interest.

10. The method according to claim 9, wherein displaying data on the 3D display device is only performed when an operator is unable to resolve the detected threat objects from inspecting the data displayed on the 2D display device.

11. A system for screening luggage comprising:

    - a CT scanner to generate volumetric image data of luggage to be screened;
    - a 2D display workstation for an operator to visualize said volumetric image data to perform visual analysis of scanned luggage; and
    - a 3D display workstation for another operator to visualize said volumetric image data to perform visual analysis of scanned luggage only when said operator can not resolve the scanned luggage using said 2D display workstation within a predetermined time period.

12. The system according to claim 11, wherein said 3D display workstation is further used to assist operators to locate objects when opening and searching a suspected bag.

13. The system according to claim 11, wherein said 2D display workstation and 3D display workstation share one computer.

14. The system according to claim 11, wherein luggage screening includes carry-on luggage screening at checkpoints of airports.

FIG. 1

Prior Art

FIG. 2
Prior Art

FIG. 3
Prior Art

420

Display System

450

Input Device

400

Image Data and
Automatic Threat
Detection Results
Generation

Data
Processing
Device

Bag
Manipulation
Functions

454

Bag Resolution
Functions

440

452

2D Display

444

Operator

460

PRIOR ART
FIG. 4

Monitor with
right eye image

520

540

Right Eye

45°

135°

45°

Left Eye

45°

Passive Polarizing
Glasses

Monitor with
left eye image

Beamsplitter
Mirror

510

530

PRIOR ART

FIG. 5

FIG. 6

EP 2 309 257 A1

FIG. 7

802

804

Index Image

Look-Up-Table

812

Generating projection of
index image

814

Performing edge
detection

820

Mapping edge points to
3D contour volume

821

Generating contour highlighted RGBA volume

822

Left eye
position

Generating left eye image

Generating right eye image

824

Right eye
position

826

828

FIG. 8

FIG. 9

FIG. 10

Europäisches
Patentamt

European
Patent Office

Office européen
des brevets

# EUROPEAN SEARCH REPORT

Application Number

EP 10 01 5838

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 9 282443 A (HITACHI MEDICAL CORP) 31 October 1997 (1997-10-31) * abstract * * figure 1 * ----- | 1-14 | INV. G01N23/04 G01V5/00 A61B6/03 |
| X | US 2007/168467 A1 (HU HUI [US] ET AL) 19 July 2007 (2007-07-19) * the whole document * ----- | 1-14 | |
| X | GB 2 360 685 A (UNIV NOTTINGHAM TRENT [GB]) 26 September 2001 (2001-09-26) * the whole document * ----- | 1-14 | |

| | TECHNICAL FIELDS SEARCHED (IPC) |
|---|---|
| | G01N G01V A61B |

The present search report has been drawn up for all claims

| Place of search | Date of completion of the search | Examiner |
|---|---|---|
| Munich | 21 February 2011 | Rouault, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 10 01 5838

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2011

| Patent document cited in search report | | Publication date | Patent family member(s) | Publication date |
|---|---|---|---|---|
| JP 9282443 | A | 31-10-1997 | NONE | |
| US 2007168467 | A1 | 19-07-2007 | NONE | |
| GB 2360685 | A | 26-09-2001 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 83155897 A, Gregory L. Larson
- US 5802134 A
- US 94893097 A, Andrew P. Tybinkowski
- US 5982844 A
- US 94893797 A, David A. Schafer
- US 5949842 A
- US 94892897 A, Christopher C. Ruth
- US 5970113 A
- US 94849197 A, Christopher C. Ruth
- US 5909477 A
- US 94892997 A, Christopher C. Ruth
- US 5901198 A
- US 94869797 A, Christopher C. Ruth
- US 5887047 A
- US 94849297 A, Christopher C. Ruth
- US 5881122 A
- US 94912797 A, Bernard M. Gordon
- US 6256404 B
- US 94845097 A, David A Schafer
- US 6091795 A
- US 94869297 A, Eric Bailey
- US 5982843 A
- US 94849397 A, Geoffrey A. Legg
- US 5932874 A
- US 94869897 A, Andrew P. Tybinkowski
- US 5937028 A
- US 02218998 A, Muzaffer Hiraoglu
- US 6111974 A
- US 02178198 A, Sergey Simanovsky
- US 6075871 A
- US 02206098 A, Ibrahim M. Bechwati
- US 6128365 A
- US 02216598 A, Sergey Simanovsky
- US 6025143 A
- US 02178298 A, Ibrahim M. Bechwati
- US 6076400 A
- US 02235498 A, Ibrahim M. Bechwati
- US 6108396 A
- US 02188998 A, Sergey Simanovsky
- US 6078642 A
- US 02206498 A, Muzaffer Hiraoglu
- US 6026171 A
- US 02206298 A, Muzaffer Hiraoglu
- US 6272230 B
- US 02216498 A, Muzaffer Hiraoglu
- US 6035014 A
- US 02220498 A, Sergey Simanovsky
- US 6067366 A
- US 22838099 A, Sergey Simanovsky
- US 6195444 B
- US 02205998 A, Sergey Simanovsky
- US 6317509 B
- US 22837999 A, Carl R. Crawford
- US 6345113 B
- US 12146602 A, Ibrahim M. Bechwati
- US 6687326 B
- US 17111602 A, Ram Naidu
- US 6721387 B
- US 98219201 A, John M. Dobbs
- US 6748043 B
- US 84207501 A, Seemeen Karimi
- US 6813374 B
- US 61157203 A, Ram Naidu
- US 7197172 B
- US 83190904 A, Zhengrong Ying
- US 7277577 B
- US 85091004 A, Zhengrong Ying
- US 7190757 B
- US 85394204 A, Zhengrong Ying
- US 7136450 B
- US 86098404 A, Zhengrong Ying
- US 86461904 A, Zhengrong Ying
- US 7327853 B
- US 88319904 A, Gregory L. Larson
- US 7302083 B
- US 89977504 A, Ram Naidu
- US 7224763 B
- US 92063504 A, Anton Deykoon
- US 95871304 A, Ram Naidu
- US 7136451 B
- US 14221605 A, Zhengrong Ying
- US 18347105 A, Zhengrong Ying
- US 18337805 A, Sergey Simanovsky
- US 19836005 A, Ram Naidu
- US 70448207 A, Zhengrong Ying
- US 76937007 A, Ram Naidu
- US 6554430 B, Dorval

**Non-patent literature cited in the description**

- **FERGASON et al.** An innovative beamsplitter-based stereoscopic 3-D display design. *Proc SPIE 5664,* 2005, 488